# NEUE EUROPÄISCHE PATENTSCHRIFT

(11) **EP 0 858 796 B2**
(45) Veröffentlichungstag und Bekanntmachung des Hinweises auf die Entscheidung über den Einspruch: **11.04.2007**
(45) Hinweis auf die Patenterteilung: 26.02.2003
(21) Anmeldenummer: 97118996.4
(22) Anmeldetag: 31.10.1997
(51) Int. Cl.: A61K 8/22, A61K 8/41, A61Q 5/10

(54) **Mittel zum oxidativen Färben von Haaren**
Oxidative hair dye
Composition de teinture d'oxydation des cheveux

(30) Priorität: 15.02.1997 DE 19705875
(43) Veröffentlichungstag der Anmeldung: 19.08.1998
(73) Patentinhaber: Wella Aktiengesellschaft, 64274 Darmstadt (DE)
(72) Erfinder: Döhling, Annelie, 64839 Münster (DE); Lauscher, Dirk, 64372 Ober-Ramstadt (DE)

(56) Entgegenhaltungen:
- EP-A- 0 560 088
- EP-A- 0 630 643
- EP-A- 0 815 830
- EP-A- 0 861 656
- WO-A-97/39724
- DE-A- 19 600 704
- GB-A- 1 596 956
- SCHRADER K.: "Grundlagen und Rezepturen der Kosmetik", 2. Auflage, 1989, Hüthig Buch Verlag, Seiten 784-805
- RÖMPP CHEMIE LEXIKON, 9. Auflage 1989, Seiten 346, 558, 2595, 2596

## Beschreibung

Gegenstand der vorliegenden Erfindung ist ein Mittel zum oxidativen Färben von Keratinfasern, insbesondere menschlichen Haaren, welches als Oxidationsmittel mindestens ein Erdalkaliperoxid oder eine Kombination aus mindestens einem Erdalkaliperoxid und mindestens einer anorganischen oder organischen Säuren beziehungsweise deren Salzen enthält, sowie die Verwendung von Erdalkaliperoxiden als Oxidationsmittel in Mitteln zum oxidativen Färben von Keratinfasern

Für das Färben von keratinhaltigen Fasern, zum Beispiel Wolle, Pelzen, Federn und insbesondere Humanhaaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Direktziehende Farbstoffe werden zwar unter relativ schonenden Bedingungen appliziert, jedoch besitzen direktziehende Farbstoffe nur unzureichende Echtheitseigenschaften. Mit Oxidationsfarbstoffen lassen sich in der Regel intensive Färbungen mit guten Echtheitseigenschaften erzielen, die Entwicklung der Farbe geschieht jedoch unter dem Einfluß von starken Oxidationsmitteln, wie zum Beispiel Wasserstoffperoxid, was häufig Schädigungen der Faser zur Folge hat. Aus der DE-OS 44 45 282 ist der Einsatz von leichtlöslichen Alkaliperoxiden und Alkalihyperoxiden als Oxidationsmittel bekannt, jedoch entstehen bei deren heftigen Reaktion dieser Peroxide mit Wasser die stark basischen und leichtlöslichen Alkalihydroxide, die ebenfalls schädigend auf die Keratinfaser wirken. In der DE-OS 26 28 398 wird ein Verfahren zur Färbung von Haaren beschrieben, bei dem durch Vorbehandlung der Fasern mit einer stark verdünnten Übergangsmetallsalzlösung beziehungsweise Eintragen von geringen Mengen eines Übergangsmetallsalzes in das Färbemittel die Kupplungsreaktion so aktiviert wird, daß sie auch unter schwach alkalischen Bedingungen ausreichend schnell abläuft. Allerdings reichem sich die Schwermetallionen in der Keratinfaser an, so daß bei wiederholter Behandlung der Haare Schädigungen auftreten können. Der Nachteil aller vorgenannten Oxidationsmittel liegt zusätzlich darin, daß sie zu Beginn der Anwendung in hoher Konzentration vorliegen und diese Konzentration erst im Verlauf der Haarbehandlung abnimmt. Aus der GB-PS 1 596 956 sind Färbemittel auf der Basis von 5,6-Dihydroxy-indolen bekannt, wobei die farbbildende Reaktion der Indole in Gegenwart eines Oxidationsmitteles, beispielsweise Wasserstoffperoxid oder Persulfate oder aber Bariumperoxid, erfolgt. Indole weisen jedoch in der Regel nur eine begrenzte Lagerstabilität auf, wobei die Indole in Gegenwart eines Oxidationsmittels sehr schnell polymerisieren.

Aufgabe der vorliegenden Erfindung ist es, eine oxidative Färbung von Keratinfasern zu ermöglichen, bei der die vorgenannten Nachteile nicht auftreten.

Überraschend wurde nun gefunden, daß die vorstehend bechriebenen Nachteile vermieden werden können, wenn als Oxidationsmittel für oxidative Haarfärbemittel auf der Basis mindestens einer Entwicklersubstanz aus der Gruppe bestehend aus 1,4-Diaminobenzol, 1,4-Diamino-2-methylbenzol, 1,4-Diamino-2,6-dimethylbenzol, 1,4-Diamino-2,5-dimethyl-benzol, 1,4-Diamino-2,3-dimethyl-benzol, 1,4-Diamino-2-chlor-benzol, 4-Di[(2-hydroxyethyl)amino]-anilin, 4-[(2-Methoxyethyl)amino]-anilin, 1,4-Diamino-2-(2-hydroxyethyl)-benzol, 1,3-Bis-[N(2-hydroxyethyl)-N-(4-aminophenyl)-amino-2-propanol, 2,'2-[1,2-Ethandiyl-bis(oxy-2,1-ethandiyloxy)]-bis-1,4-diamino-benzol, 4-Aminophenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraamino-pyrimidin, 2,5,6-Triamino-4-hydroxy-pyrimidin, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2,5-Dimethylpyridin, 2-Amino-6-methyl-phenol und 2-Amino-5-methyl-phenol, und mindestens einer Kupplersubstanz aus der Gruppe bestehend aus N,N-Dimethyl-3-ureido-anilin, 2,6-Diaminopyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy- ethoxy)-5-methyl-benzol, 2,4-Di[(2-hydroxyethyl)-amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 3-Di[(2-hydroxyethyl)-amino]-anilin, 4-Amino-1-ethoxy-2-di[(2-hydroxyethyl)-amino]-benzol, 5-Methyl-2-(1-methylethyl)-phenol, 3-[(2-Hydroxy-ethyl)amino]-anilin, 3-[(2-Aminoethyl)amino]-anilin, 1,3-Di(2,4-diaminophenoxy)-propan, 2,4-Dimethoxy-1,3-diamino-benzol, 2,6-Bis(2-hydroxyethyl)amino-toluol, 3-Dimethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methylphenol, 3-Amino-2,4-dichlor-phenol, 3-Diethylamino-phenol, 3-Amino-2-chlor-6-methyl-phenol, 3-Aminophenol, 3-[(Amidomethyl)amino]-phenol, 5-[(2-Hydroxyethyl)amino]-2-methyl-phenol, 3-[(2-Hydroxyethyl)amino]-phenol, 5-Amino-2-ethyl-phenol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 5-[(2-Hydroxyethyl)amino]-1,3-benzodioxol, 1,3-Dihydroxybenzol, 4-Chlor-1,3-dihydroxybenzol, 1,3-Dihydroxy-2-methyl-benzol, 3,4-Methylendioxybenzol, 3,4-Methylendioxyanilin, 1-Hydroxy-6-brom-3,4-methylendioxybenzol, 5-Amino-4-chlor-2-methyl-phenol, 3,4-Diamino-benzoesäure, 6-Hydroxy-2H-1,4-benzoxazin, 2,7-Dihydroxynaphthalin, 1-Naphthol, 1,7-Dihydroxynaphthalin, 1,5-Dihydroxynaphthalin, 2,6-Dihydroxy-4-methyl-pyridin, 2,6-Dihydroxy-pyridin, 2-Methyl-1-naphthol-acetat, Phenylmethylpyrazolon, 2,6-Dihydroxy-3,4-dimethylpyridin und 2-Amino-3-hydroxy-pyrimidin, Erdalkaliperoxide verwendet werden, wobei eine Anmischung mit einer Wasserstoffperoxidlösung unnötig wird. Erdalkaliperoxide sind schwerlösliche Verbindungen, die eine hervorragende Stabilität aufweisen.
Die Konzentration an reaktionsfähigem Erdalkaliperoxid im gebrauchsfertigen Färbemittel ist aufgrund der Schwerlöslichkeit der Verbindungen während der gesamten Anwendungszeit konstant gering.

Gegenstand der vorliegenden Anmeldung ist daher ein Mittel zum oxidativen Färben von Keratinfasern, insbesondere menschlichen Haaren, auf der Basis von Oxidationsfarbstoffvorstufen, welches dadurch gekennzeichnet ist, daß es bezogen auf das gebrauchsfertige Färbemittel 0,01 bis 25 Gewichtsprozent mindestens eines Erdalkaliperoxides, vorzugsweise Magnesiumperoxid, Calciumperoxid, Bariumperoxid und/oder Strontiumperoxid, insbesondere Calciumperoxid und/oder Bariumperoxid, enthält.

Weiterhin wurde überraschend gefunden, daß durch Zusatz von anorganischen oder organischen Säuren zu dem mindestens ein Erdalkaliperoxid enthaltenden Färbemittel die Farbintensität deutlich verbessert wird.

Ein weiterer Gegenstand der vorliegenden Anmeldung ist daher ein Mittel zum oxidativen Färben von Keratinfasern, insbesondere menschlichen Haaren, auf der Basis mindestens einer Entwicklersubstanz aus der Gruppe bestehend aus 1,4-Diaminobenzol, 1,4-Diamino-2-methylbenzol, 1,4-Diamino-2,6-dimethylbenzol, 1,4-Diamino-2,5-dimethyl-benzol, 1,4-Diamino-2,3-dimethyl-benzol, 1,4-Diamino-2-chlor-benzol, 4-Di[(2-hydroxyethyl)amino]-anilin, 4-[(2-Methoxyethyl)amino]-anilin, 1,4-Diamino-2-(2-hydroxyethyl)-benzol, 1,3-Bis-[N(2-hydroxyethyl)-N-(4-aminophenyl)-amino-2-propanol, 2,'2-[1,2-Ethandiyl-bis(oxy-2,1-ethandiyloxy)]-bis-1,4-diamino-benzol, 4-Aminophenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraamino-pyrimidin, 2,5,6-Triamino-4-hydroxy-pyrimidin, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2,5-Dimethylpyridin, 2-Amino-6-methyl-phenol und 2-Amino-5-methyl-phenol, und mindestens einer Kupplersubstanz aus der Gruppe bestehend aus N,N-Dimethyl-3-ureido-anilin, 2,6-Diaminopyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy- ethoxy)-5-methyl-benzol, 2,4-Di[(2-hydroxyethyl)-amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 3-Di[(2-hydroxyethyl)-amino]-anilin, 4-Amino-1-ethoxy-2-di[(2-hydroxyethyl)-amino]-benzol, 5-Methyl-2-(1-methylethyl)-phenol, 3-[(2-Hydroxy-ethyl)amino]-anilin, 3-[(2-Aminoethyl)amino]-anilin, 1,3-Di(2,4-diaminophenoxy)-propan, 2,4-Dimethoxy-1,3-diamino-benzol, 2,6-Bis(2-hydroxyethyl)amino-toluol, 3-Dimethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methylphenol, 3-Amino-2,4-dichlor-phenol, 3-Diethylamino-phenol, 3-Amino-2-chlor-6-methyl-phenol, 3-Aminophenol, 3-[(Amidomethyl)amino]-phenol, 5-[(2-Hydroxyethyl)amino]-2-methyl-phenol, 3-[(2-Hydroxyethyl)amino]-phenol, 5-Amino-2-ethyl-phenol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 5-[(2-Hydroxyethyl)amino]-1,3-benzodioxol, 1,3-Dihydroxybenzol, 4-Chlor-1,3-dihydroxybenzol, 1,3-Dihydroxy-2-methyl-benzol, 3,4-Methylendioxybenzol, 3,4-Methylendioxyanilin, 1-Hydroxy-6-brom-3,4-methylendioxybenzol, 5-Amino-4-chlor-2-methyl-phenol, 3,4-Diamino-benzoesäure, 6-Hydroxy-2H-1,4-benzoxazin, 2,7-Dihydroxynaphthalin, 1-Naphthol, 1,7-Dihydroxynaphthalin, 1,5-Dihydroxynaphthalin, 2,6-Dihydroxy-4-methyl-pyridin, 2,6-Dihydroxy-pyridin, 2-Methyl-1-naphthol-acetat, Phenylmethylpyrazolon, 2,6-Dihydroxy-3,4-dimethylpyridin und 2-Amino-3-hydroxy-pyrimidin, welches zusätzlich zu dem Erdalkaliperoxid mindestens eine anorganische oder organische Säure enthält.

Als anorganische Säuren können die folgenden Säuren genannt werden: Salzsäure, Schwefelsäure und Phosphorsäure oder deren Alkalisalzen und Ammoniumsalzen, vorzugsweise puffernden Salzen wie Natriumhydrogensulfat, Kaliumhydrogensulfat oder Ammoniumhydrogensulfat, Diammoniumsulfat, Natriumdihydrogenphosphat, Kaliumdihydrogenphosphat oder Ammoniumdihydrogenphosphat, Dinatriumhydrogenphosphat, Dikaliumhydrogenphosphat oder Diammoniumhydrogenphosphat und Ammoniumchlorid.

Als organische Säuren können genannt werden: Monocarbonsäureri wie Essigsäure, Propionsäure oder Palmitinsäure; Polycarbonsäuren und Dicarbonsäuren wie Oxalsäure, Malonsäure oder Phthalsäure; Aminosäuren wie Glycin, Alanin, Leucin oder Aminosäuren mit weiteren funktionellen Gruppen wie Serin, Cystein, Lysin, Arginin, Ornithin, Citrullin oder Carnitin; Aminopolycarbonsäuren wie Nitrilotriessigsäure, Methylglycindiessigsäure, Ethylglycindiessigsäure, β-Alanindiessigsäure, Serindiessigsäure, Isoserindiessigsäure, Asparagindiessigsäure, Polyasparaginsäure, Imidodisuccinat, Iminodiessigsäure, Methyliminodiessigsäure, Hydroxyethylethylendiamintriessigsäure, Ethylendiamintetraessigsäure oder Diethylentriaminpentaessigsäure; Phosphonsäuren wie Acetophosphonsäure, Hydroxyphosphonsäuren und Aminophosphonsäuren, insbesondere mit mehreren Phosphonsäuregruppen wie 1-Hydroxyethan-1,1-diphosphonsäure, Aminotri-(methylenphosphonsäure), Alkylendiaminotetra(methylenphosphonsäure) und Dialkylentriaminopenta(methylenphosphonsäure), wobei Alkylen vorzugsweise für Ethylen, 1,2-Propylen und 1,3-Propylen sowie 1,2-Butylen und 1,4-Butylen steht; Sulfonsäuren wie Toluolsulfonsäure; Aminosulfonsäuren wie Sulfanilsäure; Monohydroxycarbonsäuren oder Polyhydroxycarbonsäuren, insbesondere α-Hydroxycarbonsäuren, wie zum Beispiel Glykolsäure, Milchsäure, Gluconsäure und andere Zuckersäuren, Äpfelsäure, Weinsäure, Citronensäure oder Schleimsäure sowie die Alkalisalze und Ammoniumsalze dieser organischen Säuren, insbesondere puffernde Salze wie Natriumacetat, Kaliumacetat oder Ammoniumacetat, Natriumoxalat, Kaliumoxalat oder Ammoniumoxalat; die Alkalisalze der obengenannten Phosphonsäuren und Sulfonsäuren; die Alkalisalze der Hydroxyphosphonsäuren, die Alkalisalze der Aminophosphonsäuren, sowie die Alkalisalze der Aminopolycarbonsäuren, beispielsweise Diammoniumtartrat, Dinatriumtartrat, Dikaliumtartrat oder Kalium-/Natriumtartrat, Kaliumhydrogenphthalat, Kaliumhydrogentartrat, Natriumgluconat, Natriumdihydrogencitrat, Kaliumdihydrogencitrat, Ammoniumdihydrogencitrat, Dinatriumhydrogencitrat, Dikaliumhydrogencitrat, Diammoniumhydrogencitrat, Trinatriumcitrat, Trikaliumcitrat und Magnesiumcitrat sowie Kombinationen dieser Substanzen. Besonders bevorzugt sind hierbei α-Hydroxycarbonsäuren, wie zum Beispiel Citronensäure, Äpfelsäure und Weinsäure oder deren Kombinationen, sowie deren Salze.

Die vorgenannten anorganischen und organischen Säuren können sowohl alleine als auch in Kombination miteinander verwendet werden.

Durch den Säurezusatz wird sowohl das Solvolysegleichgewicht und damit die Aktivität der Erdalkaliperoxide geregelt, als auch der pH-Wert des Färbepräparates gesteuert, so daß sehr milde Bedingungen für die Anwendung an keratinischen Fasern eingestellt werden können.

Der pH-Wert der gebrauchsfertigen Färbezubereitung liegt etwa in einem Bereich von 4 bis 11, bevorzugt 7 bis 10, besonders bevorzugt 7,7 bis 9,3.

Bezogen auf das gebrauchsfertige Oxidationsfärbemittel werden die Erdalkaliperoxide in einer Gesamtmenge von 0,01 bis 25 Gewichtsprozent, vorzugsweise in einer Gesamtmenge von 0,1 bis 10 Gewichtsprozent, insbesondere in einer Gesamtmenge von 0,5 bis 5 Gewichtsprozent, eingesetzt.

Der Zusatz an anorganischen oder organischen Säuren beziehungsweise deren Salzen oder Kombinationen daraus beträgt bezogen auf das gebrauchsfertige Oxidationsfärbemittel 0,01 bis 25 Gewichtsprozent, vorzugsweise 0,1 bis 15 Gewichtsprozent, wobei eine Gesamtmenge von 0,2 bis 7,5 Gewichtsprozent besonders bevorzugt ist. Unter Verwendung des beschriebenen Oxidationsmittels insbesondere bei Verwendung einer Kombination aus Magnesiumperoxid, Calciumperoxid, Bariumperoxid und/oder Strontiumperoxid mit einer organischen und/oder anorganischen Säure lassen sich übliche Oxidationsfarbstoffvorstufen auch ohne Zusatz der sonst erforderlichen Wasserstoffperoxidlösungen beziehungsweise Wasserstoffperoxidadditionsverbindungen miteinander zu Farbstoffen kuppeln.

Das erfindungsgemäße Mittel enthält einen oder mehrere der vorgenannten Entwicklersubstanzen und Kupplersubstanzen (=Oxidationsfarbstoffvorstufen), gegebenenfalls in Kombination mit direktziehenden Farbstoffe, wobei die Farbstoffe, sofern es Basen sind, auch in Form der physiologisch verträglichen Säure-additionssalze, beispielsweise als Hydrochlorid beziehungsweise Sulfat, oder - sofern sie aromatische OH-Gruppen besitzen - in Form der Salze mit Basen, zum Beispiel als Alkaliphenolat, eingesetzt werden können.

Die Oxidationsfarbstoffvorstufen sind, bezogen auf das gebrauchsfertige Oxidationsfärbemittel, in dem erfindungsgemäßen Färbemittel in einer Gesamtmenge von 0,02 bis 20 Gewichtsprozent, vorzugsweise in einer Gesamtmenge von 0,5 bis 5 Gewichtsprozent enthalten. Die Entwicklersubstanzen und Kupplersubstanzen werden vorzugsweise in äquimolaren Mengen eingesetzt. Es ist jedoch nicht nachteilig, wenn eine dieser beiden Substanzklassen in einem gewissen Überschuß oder Unterschuß enthalten ist. So können beispielsweise der Entwickler und der Kuppler in einem Verhältnis von 1:0,5 bis 1:2 eingesetzt werden.

Zur Erzielung spezieller Farbnuancen können zusätzlich übliche direktziehende Farbstoffe, beispielsweise Triphenylmethanfarbstoffe wie Basic Violet 14 (C.I. 42 510) und Basic Violet 2(C.I. 42 520), aromatische Nitrofarbstoffe wie 2-Amino-4,6-dinitrophenol, 2-Nitro-4-(2'-hydroxyethylamino)-anilin und 2-Amino-4-nitrophenol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue (C.I. 64 500), Disperse Red 15 (C.I. 60 710), Disperse Violet 1 (C.I. 61 100), 1,4,5,8,-Tetraaminoanthrachinon und 1,4-Diaminoanthrachinon in dem erfindungsgemäßen Färbemittel enthalten sein.

Wegen der Hydrolyseempfindlichkeit können Erdalkaliperoxide nicht in wässriger Lösung aufbewahrt werden. Formulierungen, die das erfindungsgemäße Oxidationssystem enthalten, sind deshalb wasserfrei, wobei der Begriff "wasserfrei" so zu verstehen ist, daß die Gesamtmenge des in den verwendeten Rohstoffen enthaltenen Kristallwassers beziehungsweise der in den verwendeten Rohstoffen enthaltenen Feuchtigkeitsspuren in dem erfindungsgemäßen Färbemittel bezogen auf das gebrauchsfertige Färbemittel nicht größer als 10 Gewichtsprozent, vorzugsweise kleiner als 4 Gewichtsprozent insbesondere kleiner als 1 Gewichtsprozent, ist.

Das erfindungsgemäße Oxidationsmittel bestehend aus mindestens einem Erdalkaliperoxid oder aus einer Kombination aus mindestens einem Erdalkaliperoxid und mindestens einer organischen und/oder anorganischen Säure wird als Gemisch mit den Farbstoffvorstufen in einem wasserfreien Medium, vorzugsweise in Form eines staubfreien Pulvers, formuliert, wobei dieses Gemisch unmittelbar vor der Anwendung zum Starten der Färbereaktion mit Wasser, vorzugsweise in einem Gewichtsverhältnis von 1:0,5 bis 1:20, versetzt wird. Ebenfalls ist es möglich das erfindungsgemäße Oxidationshaarfärbemittel in Form einer 2-Komponenten-Zubereitung abzupacken, wobei die Erdalkaliperoxide als wasserfreie Formulierung in Pulverform, oder mikroverkapselt, oder als Suspension in einem wasserfreien Medium getrennt von den übrigen Komponenten des Oxidationshaarfärbemittels abgepackt sind, während die übrigen Komponenten des Oxidationshaar-färbemittels in Form einer wasserhaltigen Zubereitung vorliegen . Das Oxidationsmittel wird in diesem Fall unmittelbar vor der Anwendung innig mit der wasserhaltigen Zubereitung vermischt, wobei die wasserhaltige Zubereitung auch in Form eines Konzentrates vorliegen kann, das vor der Anwendung mit Wasser verdünnt wird.

Das erfindungsgemäße Oxidationssystem und die Farbstoffvorstufen werden einzeln oder als Gemisch in kosmetische Zubereitungen, welche als Trägermasse dienen, eingearbeitet, wobei sich die Erdalkaliperoxide in einem gemäß der obengenannten Definition wasserfreien Milieu befinden.

Geeignete kosmetische Zubereitungen sind beispielsweise Cremes, Emulsionen, Gele, tensidhaltige Lösungen wie Shampoos, Schaumaerosole oder andere Formulierungen, die für haarkosmetische Zwecke geeignet sind. Übliche Bestandteile solcher Zubereitungen werden in der einschlägigen Fachliteratur, beispielsweise K. Schrader, "Grundlagen und Rezepturen der Kosmetika, 2. Auflage", Hüthig Verlag, Heidelberg, 1989, beschrieben.

Als Bestandteile von kosmetischen Zubereitungen können beispielsweise genannt werden: Tenside und Emulgatoren wie anionische, nichtionische oder ampholytische oberflächenaktive Verbindungen, zum Beispiel Fettalkoholsulfate, Alkansulfonate, Olefinsulfonate, Fettalkoholpolyglykolethersulfate, Alkylpolyglycoside und ethoxylierte Fettalkohole, Fettsäuren, Alkylphenole, Sorbitanfettsäureester und Fettsäurealkanolamide, Verdicker und Gelbildner wie zum Beispiel Fettalkohole, Fettsäuren, Paraffinöle, Fettsäureester, Methylcellulosen oder Hydroxyethylcellulosen, Stärke, synthetische Polymerisate wie Polyvinylpyrrolidon (PVP) oder Polyacrylate, Biopolymere wie Alginsäure, Antioxidantien zur Stabilisierung der Farbstoffe wie zum Beispiel Ascorbinsäure, Thioglykolsäure oder Natriumsulfit, sowie Komplexbildner, Parfümöle und haarpflegende Zusätze wie kationische Harze, Lanolinderivate, Cholesterin, Pantothensäure, Proteinderivate und Proteinhydrolysate, Provitamine und Vitamine, sowie Pflanzenextrakte.

Die Bestandteile der kosmetischen Zubereitungen werden zur Herstellung der erfindungsgemäßen Haarfärbemittel in für diesen Zweck üblichen Mengen eingesetzt; beispielsweise die Emulgiermittel in Konzentrationen von 0,2 bis 30 Gewichtsprozent und die Verdickungsmittel in Konzentrationen von 0,1 bis 25 Gewichtsprozent, jeweils bezogen auf das gebrauchsfertige Oxidationshaarfärbemittel.

Die Anwendung des erfindungsgemäßen Haarfärbemittels erfolgt, indem man unmittelbar vor dem Gebrauch die einzelnen Komponenten miteinander vermischt, gegebenfalls mit Wasser versetzt und sodann eine für die Haarfärbung ausreichende Menge, vorzugsweise 30 bis 150 Gramm, des erhaltenen Haarfärbemittels auf das Haar aufträgt.

Die Anwendungstemperatur liegt in einem Bereich von 10 bis 50 °C, bevorzugt 20 bis 40 °C, wobei der Färbevorgang durch das Einwirken von Wärme während der Behandlung beschleunigt werden kann. Nach einer Einwirkungszeit von 10 bis 60 Minuten, vorzugsweise 30 bis 45 Minuten, insbesondere 30 Minuten bei 40 °C und 40 Minuten bei 20 °C, wird das Haarfärbemittel mit Wasser gründlich ausgespült, das Haar gegebenenfalls mit einem Shampoo gewaschen und sodann getrocknet. Das Nachwaschen mit einem Shampoo entfällt, wenn eine stark tensidhaltige kosmetische Zubereitung, beispielsweise ein Färbeshampoo verwendet wurde.

Das erfindungsgemäße Oxidationshaarfärbemittel ermöglicht eine äußerst milde und schonende Färbung der Haare, die auch ohne die Zugabe von Wasserstoffperoxid zu hervorragenden Färbeergebnissen führt.

Die folgenden Beispiele sollen den Gegenstand der Erfindung näher erläutern.

### Beispiele

### Beispiele 1 - 40: Haarfärbelösung

| | |
|---|---|
| 0,0025 mol | Entwickler gemäß Tabelle 1 |
| 0,0025 mol | Kuppler gemäß Tabelle 1 |
| 0,3 g | Dinatriumethylendiaminotetraessigsäure |
| 0,3 g | Ascorbinsäure |
| 10,0 g | Ethanol |
| 10,0 g | Laurylalkohol-diglykolethersulfat-Natriumsalz (28%ige wässrige Lösung) |
| ad 100,0 g | Wasser |

### Beispiele 1 - 24: Durchführung der Haarfärbung

Unmittelbar vor dem Gebrauch des Färbemittels werden 100 g der vorstehenden Haarfärbelösung mit einer Mischung aus 0,32 g Calciumperoxid, 0,12 g Calciumhydroxid, 0,06 g Calciumcarbonat und X g Säure beziehungsweise Salz (siehe Tabelle 1) innig vermischt. Das erhaltene gebrauchsfertige Haarfärbemittel wird auf das Haar aufgetragen und nach einer Einwirkungszeit von 30 Minuten bei 40 °C mit Wasser gründlich ausgespült. Anschließend wird das Haar getrocknet. Die resultierenden Färbungen auf gebleichtem Haar sind mit den jeweiligen pH-Werten in Tabelle 1 zusammengefaßt.

### Beispiele 25-33: Durchführung der Haarfärbung

Unmittelbar vor dem Gebrauch des Färbemittels werden 100 g der vorstehenden Haarfärbelösung mit einer Mischung aus 0,7 g Bariumperoxid und X g Säure beziehungsweise Salz (siehe Tabelle 1) innig vermischt. Das erhaltene gebrauchsfertige Haarfärbemittel wird auf das Haar aufgetragen und nach einer Einwirkungszeit von 30 Minuten bei 40 °C mit Wasser gründlich ausgespült. Anschließend wird das Haar getrocknet. Die resultierenden Färbungen auf gebleichtem Haar sind mit den jeweiligen pH-Werten in Tabelle 1 zusammengefaßt.

### Beispiele 34 - 40: Durchführung der Haarfärbung

Unmittelbar vor dem Gebrauch des Färbemittels werden 100 g der vorstehenden Haarfärbelösung mit einer Mischung aus 0,25 g Magnesiumperoxid, 0,68 g Magnesiumoxid und X g Säure beziehungsweise Salz (siehe Tabelle 1) innig vermischt. Das erhaltene gebrauchsfertige Haarfärbemittel wird auf das Haar aufgetragen und nach einer Einwirkungszeit von 30 Minuten bei 40°C mit Wasser gründlich ausgespült. Anschließend wird das Haar getrocknet. Die resultierenden Färbungen auf gebleichtem Haar sind mit den jeweiligen pH-Werten in Tabelle 1 zusammengefaßt.

**Tabelle 1**

| **Beispiel** | **Entwickler** | **Kuppler** | **Erdalkaliperoxide** | **Säuren bzw. Salze** | **pH-Wert** | **Farbe** |
|---|---|---|---|---|---|---|
| 1 | 2,3,4,6-Tetraaminopyrimidin | 2-Amino-4-(2-hydroxyethyl)-aminoanisol | CaO₂ | 2g Diammoniumtartrat | 8-8,5 | blau |
| 2 | 2,3,4,6-Tetraaminopyrimidin | 2-(2,4-Diaminophenoxy)-ethanol | CaO₂ | 2g Diammoniumtartrat | 8-8,5 | blau |
| 3 | 2,3,4,6-Tetraaminopyrimidin | 2-Hydroxy-4-aminotoluol | CaO₂ | 2g Diammoniumtartrat | 8-8,5 | violett |
| 4 | 2,3,4,6-Tetraaminopyrimidin | 1-Naphthol | CaO₂ | 2g Diammoniumtartrat | 8 - 8,5 | türkis |
| 5 | 2,3,4,6-Tetraaminopyrimidin | 2-Methylresorcin | CaO₂ | 2g Diammoniumtartrat | 8 - 8,5 | rot |
| 6 | 4,5-Diamino-1-(2-hydroxyethyl)-pyrazol | 2-Amino-4-(2-hydroxyethyl)-aminoanisol | CaO₂ | 2g Carnitinhydrochlorid | 4 - 5 | violettrot |
| 7 | 4,5-Diamino-1-(2-hydroxyethyl)-pyrazol | 3-Aminophenol | CaO₂ | 2g Carnitinhydrochlorid | 4-5 | rot |
| 8 | 4,5-Diamino-1-(2-hydroxyethyl)-pyrazol | 2-Hydroxy-4-aminotoluol | CaO₂ | 2g Carnitinhydrochlorid | 4-5 | orangerot |
| 9 | 4,5-Diamino-1-(2-hydroxyethyl)-pyrazol | N-(3-Dimethylamino)-phenylharnstoff | CaO₂ | 2g Carnitinhydrochlorid | 4-5 | blau |
| 10 | 2,5-Diaminotoluol | 1,3-Diaminobenzol | CaO₂ | 0,5g Citronensäure | 5-7 | dunkelblau |
| 11 | 2,5-Diaminotoluol | 2-Amino-4-(2-hydroxyethyl)-aminoanisol | CaO₂ | 0,5g Citronensäure | 5-7 | dunkelblau |
| 12 | 2,5-Diaminotoluol | 2-(2,4-Diaminophenoxy)-ethanol | CaO₂ | 0,5g Citronensäure | 5-7 | blau |
| 13 | 2,5-Diaminotoluol | 3-Aminophenol | CaO₂ | 0,5g Citronensäure | 5 - 7 | rotbraun |
| 14 | 2,5-Diaminotoluol | 4-(2-Hydroxyethylamino)-1,2-methylen-dioxybenzol | CaO₂ | 0,5g Citronensäure | 5 - 7 | matt gelbbraun |
| 15 | 2,5-Diaminotoluol | 2-Hydroxy-4-aminotoluol | CaO₂ | 0,5g Citronensäure | 5 - 7 | violett |
| 16 | 2,5-Diaminotoluol | 1-Naphthol | CaO₂ | 0,5g Citronensäure | 5 - 7 | blau |
| 17 | 2,5-Diaminotoluol | 2-Methylresorcin | CaO₂ | 0,5g Citronensäure | 5 - 7 | rotbraun |
| 18 | 2,5-Diaminotoluol | N-(3-Dimethylamino)-phenylharnstoff | CaO₂ | 0,5g Citronensäure | 5 - 7 | blaugrün |
| 19 | 2,5-Diaminotoluol | 2-Amino-4-(2-hydroxyethyl)-aminoanisol | CaO₂ | 5g Ammoniumchlorid + 2g Glycolsäure | 3-4 | blau |
| 20 | 2,5-Diaminotoluol | 2-Hydroxy-4-aminotoluol | CaO₂ | 5g Ammoniumchlorid + 2g Glycolsäure | 3 - 4 | orangerot |
| 21 | 2,5-Diaminotoluol | 2-Amino-4-(2-hydroxyethyl)-aminoanisol | CaO₂ | 5g Ammoniumchlorid + 1g Magnesiumcitrat | 7 - 7,5 | intensiv blau |
| 22 | 2,5-Diaminotoluol | 2-Hydroxy-4-aminotoluol | CaO₂ | 5g Ammoniumchlorid + 1g Magnesiumcitrat | 7 - 7,5 | violett |
| 23 | 2,5-Diaminotoluol | 2-Amino-4-(2-hydroxyethyl)-aminoanisol | CaO₂ | 3g Ammoniumchlorid | 8,2 | dunkelblau |
| 24 | 2,5-Diaminotoluol | 2-Hydroxy-4-aminotoluol | CaO₂ | 3g Ammoniumchloridl | 8,2 | violett |
| 25 | 2-(2-Hydroxyethyl)-1,4-diaminobenzol | 1,3-Diaminobenzol | BaO₂ | 3g Glycin | 8 - 8,5 | blau |
| 26 | 2-(2-Hydroxyethyl)-1,4-diaminobenzol | 2-Amino-4-(2-hydroxyethyl)-aminoanisol | BaO₂ | 3g Glycin | 8-8,5 | blau |
| 27 | 2-(2-Hydroxyethyl)-1,4-diaminobenzol | 2-(2,4-Diaminophenoxy)-ethanol | BaO₂ | 3g Glycin | 8-8,5 | blau |
| 28 | 2-(2-Hydroxyethyl)-1,4-diaminobenzol | 3-Aminophenol | BaO₂ | 3g Glycin | 8-8,5 | rotbraun |
| 29 | 2-(2-Hydroxyethyl)-1,4-diaminobenzol | 4-(2-Hydroxyethylamino)-1,2-methylen-dioxybenzol | BaO₂ | 3g Glycin | 8-8,5 | matt gelbbraun |
| 30 | 2-(2-Hydroxyethyl)-1,4-diaminobenzol | 2-Hydroxy-4-aminotoluol | BaO₂ | 3g Glycin | 8-8,5 | rolviolett |
| 31 | 2-(2-Hydroxyethyl)-1,4-diaminobenzol | 1-Naphthol | BaO₂ | 3g Glycin | 8-8,5 | blau |
| 32 | 2-(2-Hydroxyethyl)-1,4-diaminobenzol | 2-Methylresorcin | BaO₂ | 3g Glycin | 8 - 8,5 | rotbraun |
| 33 | 1,4-Diaminobenzol | 1,3-Diaminobenzol | MgO₂ | 2g Essigsäure | 8,5 - 9^{a)} | dunkel grünblau |
| 34 | 1,4-Diaminobenzol | 2-Amino-4-(2-hydroxyethyl)-aminoanisol | MgO₂ | 2g Essigsäure | 8,5 - 9^{a)} | dunkelblau |
| 35 | 1,4-Diaminobenzol | 2-(2,4-Diaminophenoxy)-ethanol | MgO₂ | 2g Essigsäure | 8,5 - 9^{a)} | dunkelblau |
| 36 | 1,4-Diaminobenzol | 1-Naphthol | MgO₂ | 2g Essigsäure | 8,5 - 9^{a}) | blau |
| 37 | 1,4-Diaminobenzol | 1,3-Diaminobenzol | MgO₂ | 2g Essigsäure | 4,5 | graublau |
| 38 | 1,4-Diaminobenzol | 2-Amino-4-(2-hydroxyethyl)-aminoanisol | MgO₂ | 2g Essigsäure | 4,5 | blau |
| 39 | 1,4-Diaminobenzol | 2-(2,4-Diaminophenoxy)-ethanol | MgO₂ | 2g Essigsäure | 4,5 | blau |
| 40 | 1,4-Diaminobenzol | 1-Naphthol | MgO₂ | 2g Essigsäure | 4,5 | violettblau |

| | | | | | | |
|---|---|---|---|---|---|---|
| a) pH-Wert mit 25%iger Ammoniaklösung eingestellt | | | | | | |

### Beispiel 41: Färbemittel in Gelform

| **Komponente A** | |
|---|---|
| 1,1 g | 2,5-Diaminotoluol |
| 1,2 g | 4-Amino-3-methylphenol |
| 1,2 g | 5-Amino-2-methylphenol |
| 1,4 g | 2-Amino-4-(2-hydroxyethyl)-aminoanisol |
| 0,3 g | Ascorbinsäure |
| 7,0 g | Propanol-2 |
| 15,0 g | Ölsäure |
| 10,0 g | Ammoniak (25%ige wässrige Lösung) |
| 62,8 g | Wasser |
| 100,0 g | |

| **Komponente B** | |
|---|---|
| 32,0 g | Calciumperoxid |
| 12,0 g | Calciumhydroxid |
| 6,0 g | Calciumcarbonat |
| 50.0 g | Diammoniumhydrogencarbonat |
| 100,0 g | |

50 g der Komponente A werden unmittelbar vor dem Gebrauch mit 5,0 g der Komponente B versetzt und mit 50 ml Wasser innig vermischt. Das so erhaltene gebrauchsfertige Haarfärbemittel wird auf das Haar aufgetragen und nach einer Einwirkungszeit von 30 Minuten bei 40 °C mit Wasser gründlich ausgespült. Anschließend wird das Haar getrocknet. Das Haar hat eine intensive dunkelviolette Färbung erhalten.

### Beispiel 42 - 64: Färbemittel in Cremeform

| **Komponente A** | |
|---|---|
| X g | Farbstoffvorstufen und direktziehende Farbstoffe (gemäß Tabelle 2) |
| 0,3 g | Natriumsulfit |
| 0,1 g | Ascorbinsäure |
| 3,5 g | Laurylalkoholdiglykolethersulfat-Natriumsalz (28%ige wässrige Lösung) |
| 15,0 g | Cetylalkohol |
| 3,0 g | Ammoniak (25%ige wässrige Lösung) |
| ad 100,0 g | Wasser |

| **Komponente B** | |
|---|---|
| 32,0 g | Calciumperoxid |
| 12,0 g | Calciumhydroxid |
| 6,0 g | Calciumcarbonat |
| 50.0 g | Diammoniumhydrogencarbonat |
| 100,0 g | |

50 g der Komponente A werden unmittelbar vor dem Gebrauch mit 5,0 g der Komponente B versetzt und mit 50 ml Wasser innig vermischt. Die so erhaltene Färbemasse läßt man 30 Minuten bei 40 °C auf blonde Naturhaare einwirken. Anschließend wird das Haar gründlich mit Wasser ausgespült und getrocknet. Auswahl und Menge X (in Gramm) an Farbstoffvorstufen sowie die resultierende Haarfarbe sind in Tabelle 2 angegeben.

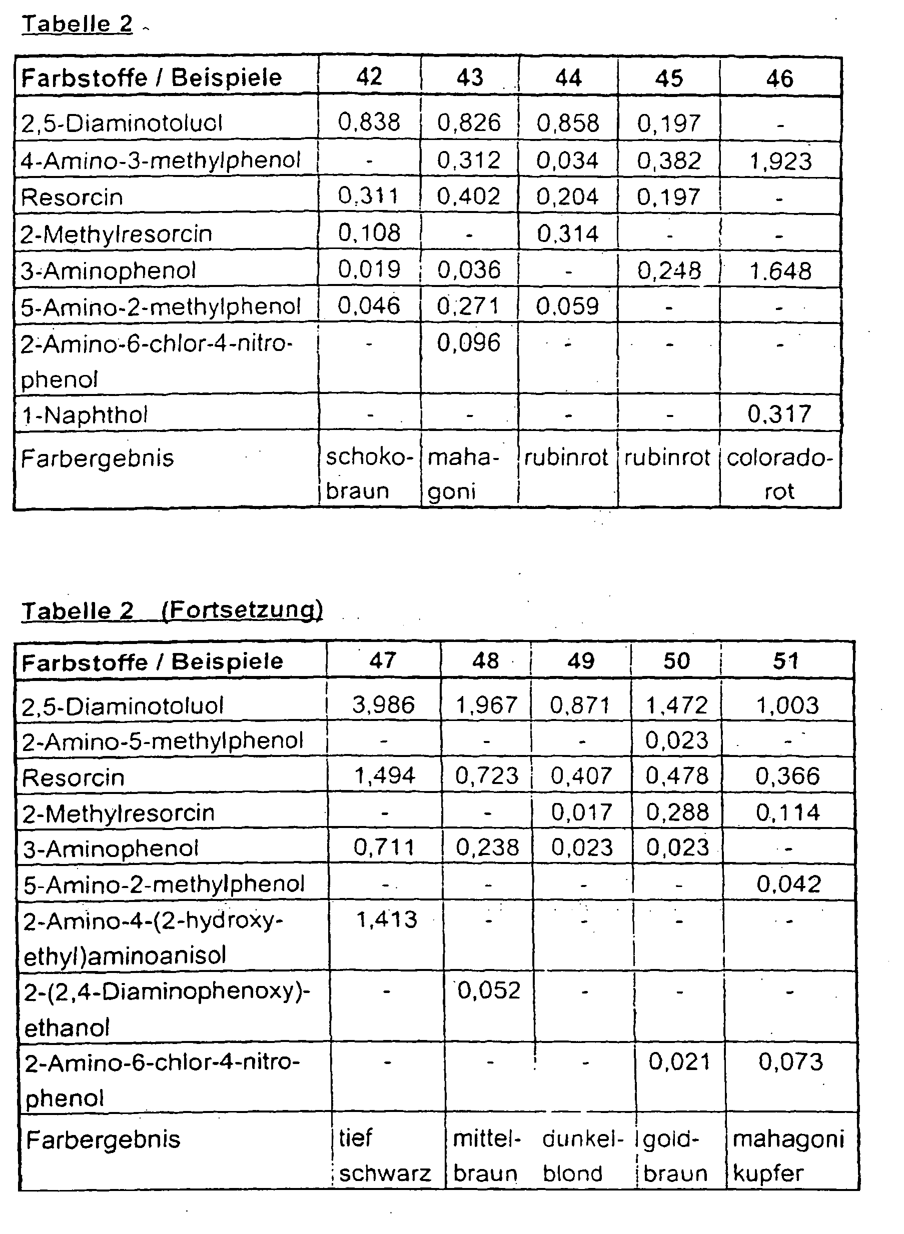

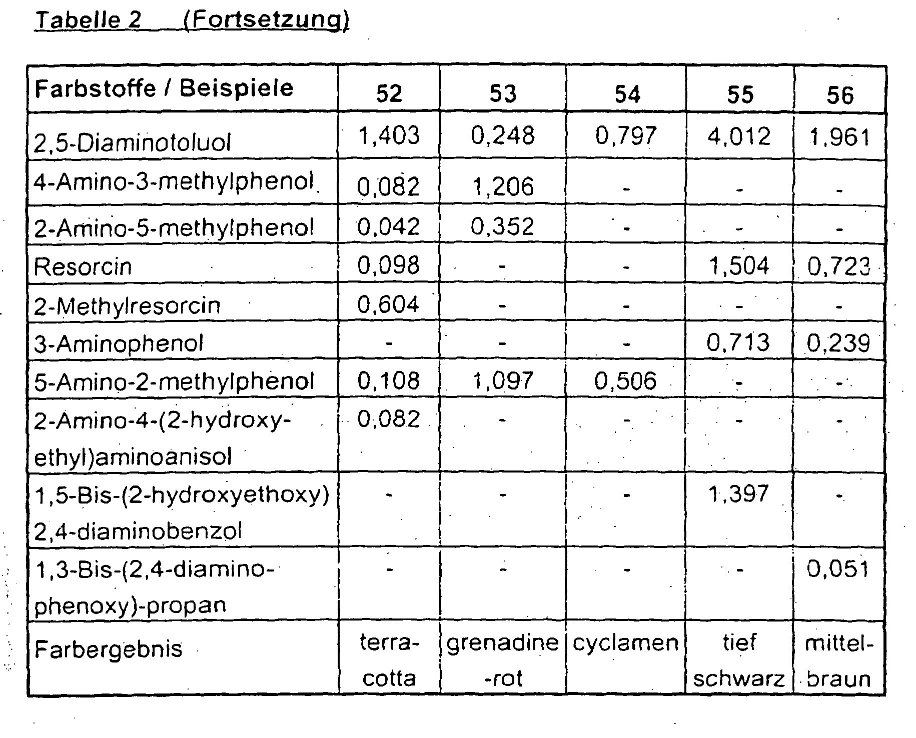

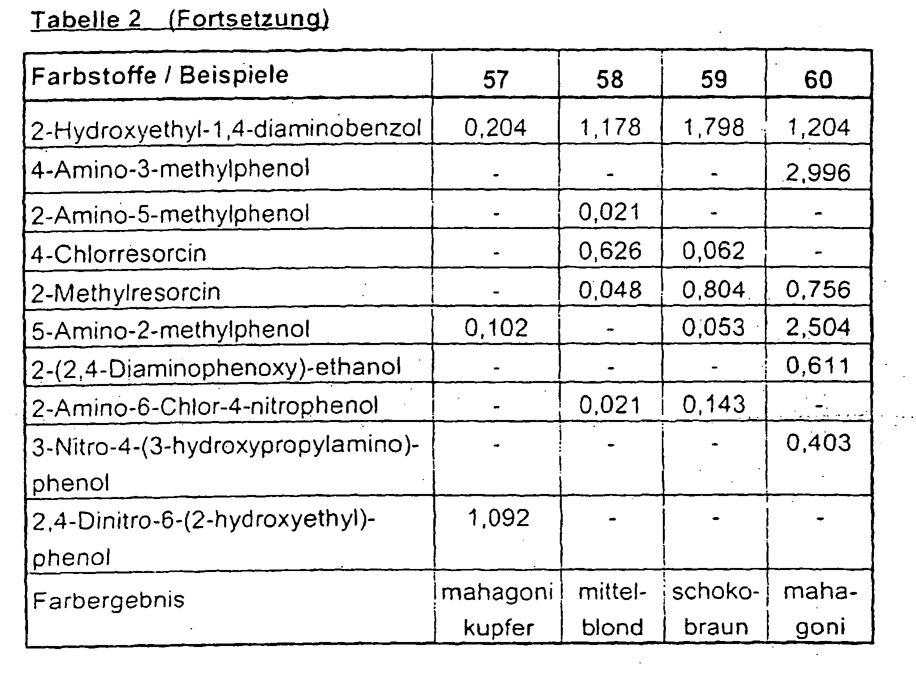

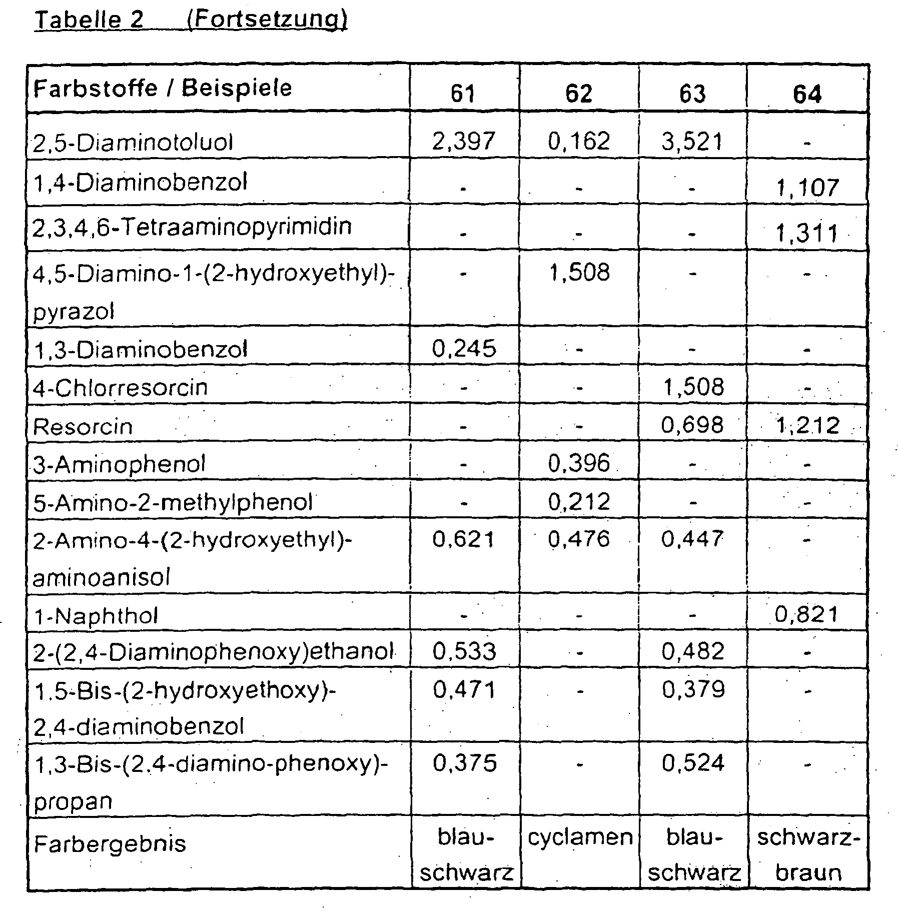

### Beispiel 65: Färbemittel in Pulverform

| | |
|---|---|
| 17,0 g | Hydroxypropylcelfulose |
| 12,8 g | Calciumperoxid |
| 10,3 g | Natriumsulfat |
| 10,0 g | Ammoniumchlorid |
| 10,0 g | Magnesiumcarbonat |
| 10,0 g | Citronensäure |
| 5,0 g | Natriumsulfit |
| 5,0 g | Siliciumdioxid |
| 4,8 g | Calciumhydroxid |
| 3,3 g | 1,4-Diaminobenzol |
| 2,0 g | Natriumlaurylsulfat |
| 2,0 g | Dinatrium-EDTA |
| 2,8 g | 2-Amino-(2-hydroxyethyl)-aminoanisol |
| 2,4 g | Calciumcarbonat |
| 1,5 g | 4-Aminophenolhydrochlorid |
| 1,1 g | Resorcin |
| 100,0 g | |

11 g dieses Färbemittels werden unmittelbar vor dem Gebrauch mit 89 ml Wasser innig vermischt. Die erhaltene gebrauchsfertige Färbemasse läßt man 30 Minuten bei 40 °C auf blonde Naturhaare einwirken. Anschließend wird das Haar gründlich mit Wasser ausgespült und getrocknet. Das Haar hat eine intensive blauschwarze Färbung erhalten.

Alfe in der vorliegenden Anmeldung genannten Prozentzahlen stellen, sofern nicht anders angegeben, Gewichtsprozente dar.

## Patentansprüche

1. Mittel zum oxidativen Färben von Keratinfasern auf der Basis mindestens einer Entwicklersubstanz aus der Gruppe bestehend aus 1,4-Diaminobenzol, 1,4-Diamino-2-methylbenzol, 1,4-Diamino-2,6-dimethylbenzol, 1,4-Diamino-2,5-dimethyl-benzol, 1,4-Diamino-2,3-dimethyl-benzol, 1,4-Diamino-2-chlor-benzol, 4-Di[(2-hydroxyethyl)amino]-anilin, 4-[(2-Methoxyethyl)amino]-anilin, 1,4-Diamino-2-(2-hydroxyethyl)-benzol, 1,3-Bis-[N(2-hydroxyethyl)-N-(4-aminophenyl)-amino-2-propanol, 2,'2-[1,2-Ethandiyl-bis(oxy-2,1-ethandiyloxy)]-bis-1,4-diamino-benzol, 4-Aminophenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraamino-pyrimidin, 2,5,6-Triamino-4-hydroxy-pyrimidin, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 4,5-Diamino-1-[(4-chlorphenyl)methyl]-1H-pyrazol, 4,5-Diamino-1-methyl-pyrazol, 2,5-Dimethylpyridin, 2-Amino-6-methyl-phenol und 2-Amino-5-methyl-phenol, und mindestens einer Kupplersubstanz aus der Gruppe bestehend aus N,N-Dimethyl-3-ureido-anilin, 2,6-Diaminopyridin. 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)-amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 3-Di[(2-hydroxyethyl)-amino]-anilin, 4-Amino-1-ethoxy-2-di[(2-hydroxyethyl)-amino]-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, 2,4-Dimethoxy-1,3-diamino-benzol, 2,6-Bis(2-hydroxyethyl)amino-toluol, 3-Dimethylamino-phenol, 5-Amino-2-methylphenol, 5-Amino-4-fluor-2-methyl-phenol, 5-Amino-4-methoxy-2-methylphenol, 5-Amino-4-ethoxy-2-methyl-phenol, 3-Amino-2,4-dichlor-phenol, 3-Diethylamino-phenol, 3-Amino-2-chlor-6-methyl-phenol, 3-Aminophenol, 3-[(Amidomethyl)amino]-phenol, 5-[(2-Hydroxyethyl)amino]-2-methylphenol, 3-[(2-Hydroxyethyl)amino]-phenol, 5-Amino-2-ethyl-phenol, 5-[(3-Hydroxypropyl)amino]-2-methyl-phenol, 3-[(2,3-Dihydroxypropyl)-amino]-2-methyl-phenol, 3-[(2-Hydroxy-ethyl)amino]-2-methyl-phenol, 5-[(2-Hydroxyethyl)amino]-1,3-benzodioxol, 1,3-Dihydroxybenzol, 4-Chlor-1,3-dihydroxybenzol, 1,3-Dihydroxy-2-methyl-benzol, 3,4-Methylendioxybenzol, 3,4-Methylendioxyanilin, 1-Hydroxy-6-brom-3,4-methylendioxybenzol, 5-Amino-4-chlor-2-methyl-phenol, 3,4-Diamino-benzoesäure, 6-Hydroxy-2H-1,4-benzoxazin, 2,7-Dihydroxynaphthalin, 1-Naphthol, 1,7-Dihydroxynaphthalin, 1,5-Dihydroxynaphthalin, 2,6-Dihydroxy-4-methyl-pyridin, 2,6-Dihydroxy-pyridin, 2-Methyl-1-naphthol-acetat, Phenylmethylpyrazolon, 2,6-Dihydroxy-3,4-dimethylpyridin und 2-Amino-3-hydroxy-pyrimidin, **dadurch gekennzeichnet, daß** es bezogen auf das gebrauchsfertige Färbemittel 0,01 bis 25 Gewichtsprozent mindestens eines Erdalkaliperoxides enthält.

2. Mittel nach Anspruch 1, **dadurch gekennzeichnet, daß** das Erdalkaliperoxid ausgewählt ist aus Magnesiumperoxid, Calciumperoxid, Bariumperoxid und/oder Strontiumperoxid.

3. Mittel nach Anspruch 1 oder 2, **dadurch gekennzeichnet, daß** es mindestens eine anorganische oder organische Säure enthält.

4. Mittel nach Anspruch 3, **dadurch gekennzeichnet, daß** die anorganische Säure ausgewählt ist aus Salzsäure, Schwefelsäure und Phosphorsäure beziehungsweise deren Salzen sowie Kombinationen dieser Säuren.

5. Mittel nach Anspruch 3, **dadurch gekennzeichnet, daß** die organische Säure ausgewählt ist aus Monocarbonsäuren, Dicarbonsäuren, Polycarbonsäuren, Sulfonsäuren, Aminosäuren, Aminopolycarbonsäuren, Aminosulfonsäuren, Phosphonsäuren, Hydroxyphosphonsäuren, Aminophosphonsäuren, Monohydroxycarbonsäuren und Polyhydroxycarbonsäuren beziehungsweise deren Salzen sowie Kombinationen dieser Säuren.

6. Mittel nach Anspruch 5, **dadurch gekennzeichnet, daß** die organische Säure ausgewählt ist aus Äpfelsäure, Citronensäure und Weinsäure beziehungsweise deren Salzen sowie Kombinationen dieser Säuren.

7. Mittel nach einem der Ansprüche 1 bis 6, **dadurch gekennzeichnet, daß** es (bezogen auf das gebrauchsfertige Färbemittel) die organische oder anorganische Säure in einer Gesamtmenge von 0,01 bis 25 Gewichtsprozent enthält.

8. Mittel nach einem der Ansprüche 1 bis 7, **dadurch gekennzeichnet, daß** die gebrauchsfertige Zubereitung einen pH-Wert von 4 bis 11 aufweist.

9. Mittel nach einem der Ansprüche 1 bis 8, **dadurch gekennzeichnet, daß** es (bezogen auf das gebrauchsfertige Färbemittel) die Entwicklersubstanzen und Kupplersubstanzen in einer Menge von 0,02 bis 20 Gewichtsprozent enthält.

10. Mittel nach Anspruch 9 , **dadurch gekennzeichnet, daß** es direktziehende Farbstoffe enthält.

11. Mittel nach einem der Ansprüche 1 bis 10, **dadurch gekennzeichnet, daß** es in Form einer wasserfreien kosmetischen Zubereitung vorliegt.

12. Mittel nach einem der Ansprüche 1 bis 11, **dadurch gekennzeichnet, daß** es in Form eines Pulvers vorliegt.

13. Mittel nach Anspruch 11 oder 12, **dadurch gekennzeichnet, daß** es vor Gebrauch mit Wasser in einem Gewichtsverhältnis von 1:0,5 bis 1:20 vermischt wird.

14. Mittel nach einem der Ansprüche 1 bis 10, **dadurch gekennzeichnet, daß** es in Form einer 2-Komponenten-Zubereitung vorliegt.

15. Mittel nach Anspruch 14, **dadurch gekennzeichnet, daß** es aus einer pulverförmigen, das Erdalkaliperoxid enthaltenden wasserfreien Zubereitung und einer wäßrigen kosmetischen Zubereitung besteht.

16. Mittel nach Anspruch 14, **dadurch gekennzeichnet, daß** es aus einer das Erdalkaliperoxid enthaltenden wasserfreien Suspension und einer wäßrigen kosmetischen Zubereitung besteht.

17. Mittel nach einem der Ansprüche 1 bis 16, **dadurch gekennzeichnet, daß** es ohne Zusatz von Wasserstoffperoxid hergestellt wird.

## Claims

1. Agent for the oxidative dyeing of keratin fibres based on at least one developer substance selected from the group consisting of 1,4-diaminobenzene, 1,4-diamino-2-methylbenzene, 1,4-diamino-2,6-dimethylbenzene, 1,4-diamino-2,5-dimethylbenzene, 1,4-diamino-2,3-dimethyl-benzene, 1,4-diamino-2-chlorbenzene, 4-di[(2-hydroxyethyl)amino]-aniline, 4-[(2-methoxyethyl)amino]-aniline, 1,4-diamino-2-(2-hydroxyethyl)-benzene, 1,3-bis-[N(2-hydroxyethyl)-N-(4-aminophenyl)-amino-2-propanol, 2,'2-[1,2-ethanediyl-bis(oxy-2,1-ethanediyloxy)]-bis-1,4-diamino-benzene, 4-aminophenol, 4-amino-3-methyl-phenol, 4-methylamino-phenol, 4-amino-2-(aminomethyl)-phenol, 4-amino-2-[(2-hydroxyethyl)-amino]methyl-phenol, 4-amino-2-(methoxymethyl)-phenol, 5-amino-salicylic acid, 2,4,5,6-tetraamino-pyrimidine, 2,5,6-triamino-4-hydroxy-pyrimidine, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 4,5-diamino-1-[(4-chlorophenyl)methyl]-1H-pyrazole, 4,5-diamino-1-methyl-pyrazole, 2,5-dimethylpyridine, 2-amino-6-methyl-phenol and 2-amino-5-methyl-phenol, and at least one coupler substance selected from the group consisting of N,N-dimethyl-3-ureido-aniline, 2,6-diaminopyridine, 2-amino-4-[(2-hydroxyethyl)amino]-anisole, 2,4-diamino-1-fluoro-5-methyl-benzene, 2,4-diamino-1-methoxy-5-methyl-benzene, 2,4-diamino-1-ethoxy-5-methyl-benzene, 2,4-diamino-1-(2-hydroxy- ethoxy)-5-methyl-benzene, 2,4-di[(2-hydroxyethyl)-amino]-1,5-dimethoxy-benzene, 2,3-diamino-6-methoxy-pyridine, 3-amino-6-methoxy-2-(methylamino)-pyridine, 2,6-diamino-3,5-dimethoxy-pyridine, 3,5-diamino-2,6-dimethoxy-pyridine, 1,3-diamino-benzene, 2,4-diamino-1-(2-hydroxyethoxy)-benzene, 3-di[(2-hydroxyethyl)-amino]-aniline, 4-amino-1-ethoxy-2-di[(2-hydroxyethyl)-amino]-benzene, 5-methyl-2-(1-methylethyl)-phenol, 3-[(2-hydroxy-ethyl)amino]-aniline, 3-[(2-aminoethyl)amino]-aniline, 1,3-di(2,4-diaminophenoxy)-propane, 2,4-dimethoxy-1,3-diamino-benzene, 2,6-bis(2-hydroxyethyl)amino-toluene, 3-dimethylamino-phenol, 5-amino-2-methyl-phenol, 5-amino-4-fluoro-2-methyl-phenol, 5-amino-4-methoxy-2-methylphenol, 5-amino-4-ethoxy-2-methyl-phenol, 3-amino-2,4-dichlorophenol, 3-diethylamino-phenol, 3-amino-2-chloro-6-methyl-phenol, 3-aminophenol, 3-[(amidomethyl)amino]-phenol, 5-[(2-hydroxyethyl)amino]-2-methyl-phenol, 3-[(2-hydroxyethyl)amino]-phenol, 5-amino-2-ethyl-phenol,5-[(3-hydroxypropyl)amino]-2-methyl-phenol, 3-[(2,3-dihydroxypropyl)-amino]-2-methyl-phenol, 3-[(2-hydroxyethyl)amino]-2-methyl-phenol, 5-[(2-hydroxyethyl)amino]-1,3-benzodioxole, 1,3-Dihydroxybenzene, 4-Chloro-1,3-dihydroxybenzene, 1,3-dihydroxy-2-methyl-benzene, 3,4-methylendioxybenzol, 3,4-methylendioxyaniline, 1-hydroxy-6-bromo-3,4-methylendioxybenzene, 5-amino-4-chloro-2-methylphenol, 3,4-diaminobenzoic acid, 6-hydroxy-2H-1,4-benzoxazine, 2,7-dihydroxynaphthalene, 1-naphthol, 1,7-dihydroxynaphthalene, 1,5-dihydroxynaphthalene, 2,6-dihydroxy-4-methyl-pyridine, 2,6-dihydroxypyridine, 2-methyl-1-naphthol acetate, phenylmethylpyrazolone, 2,6-dihydroxy-3,4-dimethylpyridine and 2-amino-3-hydroxy-pyrimidine, **characterized in that**, based on the ready-to-use colorant, it comprises from 0.01 to 25 percent by weight of at least one alkaline earth metal peroxide.

2. Agent according to Claim 1, **characterized in that** the alkaline earth metal peroxide is chosen from magnesium peroxide, calcium peroxide, barium peroxide and/or strontium peroxide.

3. Agent according to Claim 1 or 2, **characterized in that** it comprises at least one inorganic or organic acid.

4. Agent according to Claim 3, **characterized in that** the inorganic acid is chosen from hydrochloric acid, sulphuric acid and phosphoric acid or salts thereof, and combinations of these acids.

5. Agent according to Claim 3, **characterized in that** the organic acid is chosen from monocarboxylic acids, dicarboxylic acids, polycarboxylic acids, sulphonic acids, amino acids, aminopolycarboxylic acids, amino-sulphonic acids, phosphonic acids, hydroxyphosphonic acids, aminophosphonic acids, monohydroxycarboxylic acids and polyhydroxycarboxylic acids or salts thereof, and combinations of these acids.

6. Agent according to Claim 5, **characterized in that** the organic acid is chosen from malic acid, citric acid and tartaric acid or salts thereof, and combinations of these acids.

7. Agent according to one of Claims 1 to 6, **characterized in that**, based on the ready-to-use colorant, it comprises the organic or inorganic acid in a total amount of from 0.01 to 25 per cent by weight..

8. Agent according to one of Claims 1 to 7, **characterized in that** the ready-to-use preparation has a pH of from 4 to 11.

9. Agent according to one of Claims 1 to 8, **characterized in that**, based on the ready-to-use colorant, it comprises the developer and coupler substances in an amount of from 0.02 to 20 per cent by weight.

10. Agent according to Claim 9, **characterized in that** it comprises direct dyes.

11. Agent according to one of Claims 1 to 10, **characterized in that** it is in the form of an anhydrous cosmetic preparation.

12. Agent according to one of Claims 1 to 11, **characterized in that** it is in the form of a powder.

13. Agent according to Claim 11 or 12, **characterized in that**, prior to use, it is mixed with water in a weight ratio of from 1:0.5 to 1:20.

14. Agent according to one of Claims 1 to 10, **characterized in that** it is in the form of a 2-component preparation.

15. Agent according to Claim 14, **characterized in that** it consists of a pulverulent anhydrous preparation containing the alkaline earth metal peroxide, and an aqueous cosmetic preparation.

16. Agent according to Claim 14, **characterized in that** it consists of an anhydrous suspension containing the alkaline earth metal peroxide, and an aqueous cosmetic preparation.

17. Agent according to one of Claims 1 to 16, **characterized in that** it is prepared without the addition of hydrogen peroxide.

## Revendications

1. Composition pour la teinture oxydante de fibres kératiniques a base d'au moins un développeur qui est choisi parmi le 1,4-diaminobenzène, le 1,4-diamino-2-méthylbenzène, le 1,4-diamino-2,6-diméthylbenzène, le 1,4-diamino-2,5-diméthylbenzène, le 1,4-diamino-2,3-diméthyl-benzène, le 1,4-diamino-2-chlorobenzène, la 4-di[(2-hydroxyéthyl)amino]-aniline, la 4-[(2-méthoxyéthyl)amino]-aniline, le 1,4-diamino-2-(2-hydroxyéthyl)-benzène, le 1,3-bis-[N(2-hydroxyéthyl)-N-(4-aminophényl)-amino-2-propanol, le 2,'2-[1,2-éthanediyl-bis(oxy-2,1-éthanediyloxy)]-bis-1,4-diaminobenzène, le 4-aminophénol, le 4-amino-3-méthyl-phénol, le 4-méthylamino-phénol, le 4-amino-2-(aminométhyl)-phénol, le 4-amino-2-[(2-hydroxyéthyl)-amino]méthyl-phénol, le 4-amino-2-(méthoxyméthyl)-phénol, l'acide 5-aminosalicylique, la 2,4,5,6-tétraamino-pyrimidine, la 2,5,6-triamino-4-hydroxy-pyrimidine, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl]-1H-pyrazole, le 4,5-diamino-1-[(4-chlorophényl)méthyl]-1H-pyrazole, le 4,5-diamino-1-méthyl-pyrazole, la 2,5-diméthylpyridine, le 2-amino-6-méthylphénol et le 2-amino-5-méthyl-phénol, et au moins un coupleur qui est choisi parmi la N,N-diméthyl-3-uréido-aniline, la 2,6-diaminopyridine, le 2-amino-4-[(2-hydroxyéthyl)amino]-anisole, le 2,4-diamino-1-fluoro-5-méthylbenzène, le 2,4-diamino-1-méthoxy-5-méthyl-benzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxy-pyridine, la 3-amino-6-méthoxy-2-(méthylamino)-pyridine, la 2,6-diamino-3,5-diméthoxy-pyridine, la 3,5-diamino-2,6-diméthoxypyridine, le 1,3-diaminobenzène, le 2,4-diamino-1-(2-hydroxyéthoxy)-benzène, la 3-di[(2-hydroxyéthyl)amino]-aniline, le 4-amino-1-éthoxy-2-di[(2-hydroxyéthyl)amino]-benzène, le 5-méthyl-2-(1-méthyléthyl)-phénol, la 3-[(2-hydroxyéthyl)amino]-aniline, la 3-[(2-aminoéthyl)amino]-aniline, le 1,3-di(2,4-diaminophénoxy)-propane, le 2,4-diméthoxy-1,3-diaminobenzène, le 2,6-bis(2-hydroxyéthyl)aminotoluène, le 3-diméthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 3-diéthylaminophénol, le 3-amino-2-chloro-6-méthylphénol, le 3-aminophénol, le 3-[(amidométhyl)amino]-phénol, le 5-[(2-hydroxyéthyl)amino]-2-méthylphénol, le 3-[(2-hydroxyéthyl)amino]-phénol, le 5-amino-2-éthylphénol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, le 5-[(2-hydroxyéthyl)amino]-1,3-benzodioxol, le 1,3-dihydroxybenzène, le 4-chloro-1,3-dihydroxybenzène, le 1,3-dihydroxy-2-méthylbenzène, le 3,4-méthylènedioxybenzène, la 3,4-méthylènedioxyaniline, le 1-hydroxy-6-bromo-3,4-méthylènedioxybenzène, le 5-amino-4-chloro-2-méthylphénol, l'acide 3,4-diaminobenzoique, la 6-hydroxy-2H-1,4-bensoxazine, le 2,7-dihydroxynaphthalène, le 1-naphthol, le 1,7-dihydroxynaphthalène, le 1,5-dihydroxynaphthalène, la 2,6-dihydroxy-4-méthylpyridine, la 2,6-dihydroxypyridine, l'acétate 2-méthyl-1-naphthol, la phénylméthylpyrazolone, la 2,6-dihydroxy-3,4-diméthylpyridine et la 2-amino-3-hydroxypyrimidine,
**caracterisée en ce qu**'elle contient, par rapport au colorant prêt à l'emploi, de 0,01 à 25 % en poids d'au moins un peroxyde de métal alcalino-terreux.

2. Composition selon la revendication 1, **caractérisée en ce que** le peroxyde de métal alcalino-terreux est choisi parmi le peroxyde de magnésium, le peroxyde de calcium, le peroxyde de baryum et/ou le peroxyde de strontium.

3. Composition selon la revendication 1 ou 2, **caractérisée en ce qu'**elle contient au moins un acide organique ou minéral.

4. Composition selon la revendication 3, **caractérisée en ce que** l'acide minéral est choisi parmi l'acide chlorhydrique, l'acide sulfurique et l'acide phosphorique ou leurs sels, ainsi que des associations de ces acides.

5. Composition selon la revendication 3, **caractérisée en ce que** l'acide organique est choisi parmi les acides monocarboxyliques, les acides dicarboxyliques, les acides polycarboxyliques, les acides sulfoniques, les aminoacides, les acides aminopolycarboxyliques, les acides aminosulfoniques, les acides phosphoniques, les acides hydroxyphosphoniques, les acides aminophosphoniques, les acides monohydroxycarboxyliques et les acides polyhydroxycarboxyliques ou leurs sels, ainsi que des associations de ces acides.

6. Composition selon la revendication 5, **caractérisée en ce que** l'acide organique est choisi parmi l'acide malique, l'acide citrique et l'acide tartrique ou leurs sels, ainsi que des associations de ces acides.

7. Composition selon l'une quelconque des revendications 1 à 6, **caractérisée en ce qu'**elle contient, par rapport au colorant prêt à l'emploi, l'acide organique ou minéral en une quantité totale de 0,01 à 25 % en poids.

8. Composition selon l'une quelconque des revendications 1 à 7, **caractérisée en ce que** la préparation prête à l'emploi présente un pH de 4 à 11.

9. Composition selon l'une quelconque des revendications 1 à 8, **caractérisée en ce qu'**elle contient les développeurs et les coupleurs en une quantité de 0,02 à 20 % en poids, par rapport à la composition de teinture prête à l'emploi.

10. Composition selon la revendication 9, **caractérisée en ce qu'**elle contient des colorants directs.

11. Composition selon l'une quelconque des revendications 1 à 10, **caractérisée en ce qu'**elle est présente sous forme d'une préparation cosmétique anhydre.

12. Composition selon l'une quelconque des revendications 1 à 11, **caractérisée en ce qu'**elle est présente sous forme d'une poudre.

13. Composition selon la revendication 11 ou 12, **caractérisée en ce qu'**elle est mélangée avant l'emploi avec de l'eau en un rapport pondéral de 1:0,5 à 1:20.

14. Composition selon l'une quelconque des revendications 1 à 10, **caractérisée en ce qu'**elle est présente sous forme d'une préparation à deux composants.

15. Composition selon la revendication 14, **caractérisée en ce qu'**elle consiste en une préparation pulvérulente anhydre contenant le peroxyde de métal alcalino-terreux et une préparation cosmétique aqueuse.

16. Composition selon la revendication 14, **caractérisée en ce qu'**elle consiste en une suspension anhydre contenant le peroxyde de métal alcalino-terreux et une préparation cosmétique aqueuse.

17. Composition selon l'une quelconque des revendications 1 à 16, **caractérisée en ce qu'**elle est préparée sans addition de peroxyde d'hydrogène.
